# EUROPEAN PATENT SPECIFICATION

(11) **EP 1 241 164 B1**
(45) Date of publication and mention of the grant of the patent: **06.05.2004**
(21) Application number: 00985860.6
(22) Date of filing: 25.12.2000
(51) Int. Cl.: C07D 309/12

(54) **PROCESS FOR PREPARATION OF TETRAHYDROPYRANYLOXYAMINES**
VERFAHREN ZUR HERSTELLUNG VON TETRAHYDROPYRANYLOXYAMINEN
PROCEDE DE PREPARATION DE TETRAHYDROPYRANYLOXYAMINES

(30) Priority: 24.12.1999 JP 36848699
(43) Date of publication of application: 18.09.2002
(73) Proprietor: Mitsubishi Rayon Co., Ltd., Tokyo 108-8506 (JP)
(72) Inventor: SAKANO, Kunihiko, Mitsubishi Rayon Co., Ltd., Yokohama-shi, Kanagawa 230-0053 (JP); TAMURA, Kimio, Mitsubishi Rayon Co., Ltd., Otake-shi, Hiroshima 739-0693 (JP); URAGAKI, Toshitaka, Mitsubishi Rayon Co., Ltd., Yokohama-shi, Kanagawa 230-0053 (JP); OGURA, Kuniyoshi, Mitsubishi Rayon Co., Ltd., Yokohama-shi, Kanagawa 230-0053 (JP)
(74) Representative: HOFFMANN - EITLE
(86) International application number: PCT/JP2000/009188
(87) International publication number: WO 2001/047910

(56) References cited:
- EP-A- 0 372 891
- EP-A- 0 421 774
- EP-A1- 0 009 776
- EP-A1- 0 474 561
- M. MORI ET AL.: "Palladium-catalyzed carbonylation: A new synthesis of alpha-methylene gamma-, delta and epsilon-lactames and -lactones including bicyclic lactams of pyrrolizidine and indolizidine skeletons" J. ORG. CHEM., vol. 48, no. 22, 1983, pages 4058-4067, XP002226232
- FRYDMAN B ET AL: "1,4-Diaminobutanes From Furans: A New Synthetic Approach to Substituted Putrescines" TETRAHEDRON LETTERS, ELSEVIER SCIENCE PUBLISHERS, AMSTERDAM, NL, vol. 39, no. 27, 2 July 1998 (1998-07-02), pages 4765-4768, XP004120752 ISSN: 0040-4039
- B. RAJASHEKHAR ET AL.: "Synthesis of enantiomerically pure gamma-amino-beta-hadroxybutyric acid using malic acid as the chiral precursor" J. ORG. CHEM., vol. 50, 1985, pages 5480-5484, XP002228311
- M.-E. GOURDEL-MARTIN ET AL.: "Synthesis of norcarbovir analogues, the first examples of cyclobutene nucleosides unsubstituted at the vinylic position" J. ORG. CHEM., vol. 62, 1997, pages 2166-2172, XP002228312
- W.E. PARHAM ET AL.: "The protection of hydroxyl groups" J. AM. CHEM. SOC., vol. 70, 1948, pages 4187-4189, XP002228313
- G. F. WOODS ET AL.: "Dihydropyrane addition products" J. AM. CHEM. SOC., vol. 69, 1948, page 2246 XP002228314

## Description

The present invention relates to a production process for tetrahydropyranyloxyamines, which are highly useful as intermediates in the production of pharmaceuticals and agricultural chemicals.

This specification is based on a patent application filed in Japan (Japanese Unpublished Patent Application, No. Hei 11-368486), and the entire content of this Japanese application is incorporated by reference herein.

Tetrahydropyranyloxyamines, represented by the general formula (2) shown below, are extremely useful as intermediates in the production of pharmaceuticals and agricultural chemicals, as main materials, additives or precursors in the production of perfumes, resins and adhesives. 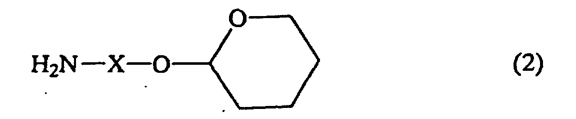 (in the formula (2), X represents a methylene group, an ethylene group or a straight chain polymethylene group having 3 to 20 carbon atoms, wherein one or more hydrogen atoms of the methylene group, ethylene group or straight chain polymethylene group having 3 to 20 carbon atoms may be substituted with either a straight chain or branched chain alkyl group, alkenyl group, alkynyl group, alkoxyalkyl group or alkylthioalkyl group, or a phenyl group or a halogen atom, and furthermore, one or more hydrogen atoms on carbon atoms not adjacent to the amino group and the hydroxyl group may be substituted with a straight chain or branched chain alkyloxy group or alkenyloxy group)

Tetrahydropyranyloxy compounds are generally formed by reacting an alcohol and 3,4-dihydro-2H-pyran in the presence of a catalytic quantity of either an inorganic acid such as hydrochloric acid or sulfuric acid or an organic acid such as p-toluenesulfonic acid.

The synthesis of one lactame described in M. Mori et al., *J*. *Org*. *Chem*., vol. 48, no 22, (1983), p. 4058-4067 proceeds via N-Benzyl-N((benzyloxy)carbonyl)-3-((tetrahydro-2H-pyran-2-yl)oxy)propylamine. This intermediate compound is prepared from a solution of 3-aminopropanol and p-toluenesulfonic acid in CH₂Cl₂ to which a solution of dihydropyran in CH₂Cl₂ is added at 0°C followed by stirring the mixture at room temperature over two hours. The solvent CH₂Cl₂ is removed under reduced pressure and the residue is dissolved in CH₃CN to which was added K₂CO₃. After further purification steps, benzylbromide in acetonitrile and later (benzyloxy)carbonylchloride are added. The total yield of this intermediate compound with respect to 3-aminopropanol was 38.9 %.

Frydman B et al. *A new Synthetic Approach to Substituted Putrescines,* vol. 39, no. 27, (1998), p. 4765-4768; B. Rajashekhar et al. *Org*. *Chem*., vol.50, (1985), p. 5480-5484; M.E. Gourdel-Martin et al. *Org*. *Chem*., vol. 62, (1997) p. 2166-2172 describe inter alia synthesis steps where the hydroxy group of organic alcohols having in addition an amino group are protected by means of the tetrahydropyranyl (THP) function. In these organic aminoalcohols, amino group and hydroxy group are not linked via a methylene group, an ethylene group or a straight chain polymethylene group having 3-20 carbon atoms. Moreover, these documents teach the use of methylene chloride as solvent for the protection reaction.

W.E. Parham et al., *J. Am. Chem. Soc*., vol. 70, (1948), p. 4187-4189; G.F. Woods et al. *J. Am. Chem. Soc*., vol. 69, (1948), p. 2246; EP-A-0 421 774; EP-A-0 372 891; EP-A1-0 009 776; EP-A1-0 474 561 describe in various contexts the protection of organic alcohols having no amino function with the THP group.

### DISCLOSURE OF INVENTION

An object of the present invention is to provide a process for producing a tetrahydropyranyloxyamine (2) from an aminoalcohol which is both simple and produces a high yield.

As a result of intensive investigations aimed at resolving problems associated with the conventional technology, the inventors of the present invention focused on the amount of acid used. The inventors then discovered that the aforementioned tetrahydropyranyloxyamine (2) could be produced in a high yield by reacting an aminoalcohol represented by the general formula (1) shown below with an acid, reacting the obtained aminoalcohol salt with 3,4-dihydro-2H-pyran to effect a tetrahydropyranylation, And then reacting the formed tetrahydropyranyloxyamine salt with an alkali under use of dimethylformamide (DMF) or dimethylsulfoxide (DMSO) as reaction solvent, and were thus able to complete the present invention.

In other words, the present invention provides a production process for a tetrahydropyranyloxyamine represented by the general formula (2) shown below, wherein an aminoalcohol represented by the general formula (1) shown below is reacted with an acid, the obtained aminoalcohol salt is then reacted with 3,4-dihydro-2H-pyran, and the formed tetrahydropyranyloxyamine salt is subsequently reacted with an alkali.

H₂N―X―OH (1)

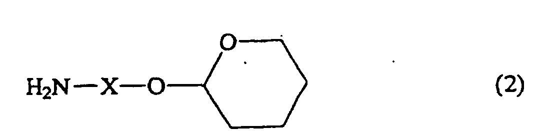

(wherein in the formula (1) and the formula (2), X represents a methylene group, an ethylene group or a straight chain polymethylene group having 3 to 20 carbon atoms, wherein one or more hydrogen atoms of the methylene group, ethylene group or straight chain polymethylene group having 3 to 20 carbon atoms may be substituted with either a straight chain or branched chain alkyl group, alkenyl group, alkynyl group, alkoxyalkyl group or alkylthioalkyl group, or a phenyl group or a halogen atom, and furthermore, one or more hydrogen atoms connected to carbon atoms not adjacent to the amino group and the hydroxyl group may be substituted with a straight chain or branched chain alkyloxy group or alkenyloxy group) wherein one of DMF or DMSO is used as reaction solvent.

### BEST MODE FOR CARRYING OUT THE INVENTION

In the aminoalcohol (1) and the tetrahydropyranyloxyamine (2), X represents a methylene group, an ethylene group or a straight chain polymethylene group having 3 to 20 carbon atoms in which one or more of the hydrogen atoms may be substituted with the groups described below. Among these groups, methylene groups, ethylene groups and straight chain polymethylene groups having 3 to 10 carbon atoms are preferred, methylene groups, ethylene groups and straight chain polymethylene groups having 3 to 6 carbon atoms are more preferred, and trimethylene groups and tetramethylene groups are the most preferred.

Examples of groups which can be used to substitute hydrogen atoms of the X group include straight chain or branched chain alkyl groups, alkenyl groups, alkynyl groups, alkoxyalkyl groups, alkylthioalkyl groups, phenyl groups, halogen atoms, alkyloxy groups and alkenyloxy groups.

In the case of substitution with an alkyl group, alkenyl group, alkynyl group, alkoxyalkyl group, alkylthioalkyl group, phenyl group, or a halogen atom, there are no particular restrictions on the position of the hydrogen atoms which can be substituted, although substitution of a hydrogen atom bonded to a secondary carbon atom is particularly preferred. In the case of substitution with an alkyloxy group or an alkenyloxy group, the hydrogen atom substituted must be bonded to a carbon atom which is not adjacent to the amino group and the hydroxy group.

Examples of preferred alkyl groups include straight chain or branched chain alkyl groups having 1 to 6 carbon atoms, straight chain or branched chain alkyl groups having 1 to 4 carbon atoms being more preferred, and methyl groups being the most preferred. Examples of preferred alkenyl groups include straight chain or branched alkenyl chain groups having 2 to 6 carbon atoms. Examples of preferred alkynyl groups include straight chain or branched chain alkenyl groups having 2 to 6 carbon atoms. Examples of preferred alkoxyalkyl groups include straight chain or branched chain alkoxyalkyl groups having 2 to 6 carbon atoms in total. Examples of preferred alkylthioalkyl groups include straight chain or branched chain alkylthioalkyl groups having 2 to 6 carbon atoms in total. Examples of preferred halogen atoms include fluorine atoms, chlorine atoms, bromine atoms and iodine atoms. Examples of preferred alkyloxy groups include straight chain or branched chain alkyloxy groups having 1 to 6 carbon atoms. Examples of preferred alkenyloxy groups include straight chain or branched chain alkenyloxy groups having 2 to 6 carbon atoms.

The most preferred X groups are trimethylene groups and 2-methyltetramethylene groups. The most preferred aminoalcohols (1) are 3-aminopropanol and 2-methyl-4-aminobutan-1-ol.

Furthermore, the most preferred tetrahydropyranyloxyamines (2) are 3-[(tetrahydro-2H-pyran-2-yl)oxy]-1-propanamine, and 3-methyl-4-[(tetrahydro-2H-pyran-2-yl)oxy]-1-butanamine.

The aminoalcohol (1) and the tetrahydropyranyloxyamine (2) may be optically active materials. Moreover, either R or S configurations are suitable. The most preferred optically active aminoalcohol (1) is (R)-2-methyl-4-aminobutan-1-ol, and the most preferred optically active tetrahydropyranyloxyamine (2) is (R)-3-methyl-4-[(tetrahydro-2H-pyran-2-yl)oxy]-1-butanamine.

Examples of suitable acids for use in the production process according to the present invention include inorganic acids such as hydrochloric acid, sulfuric acid, and phosphoric acid; organic acids such as p-toluenesulfonic acid and methanesulfonic acid; protic acids; and Lewis acids such as zinc chloride, zinc acetate, nickel chloride, aluminum chloride and tin chloride. Among these acids, in view of the improvement of the reaction yield, ease of handling, and the suppression of generation of by-products and odors, either inorganic acids such as dry hydrogen chloride gas or sulfuric acid, or organic acids such as methanesulfonic acid and p-toluenesulfonic acid are preferred, and methanesulfonic acid and p-toluenesulfonic acid are particularly preferred. By using an optically active acid, an optically active tetrahydropyranyloxyamine (2) can also be produced.

In view of improving the reaction rate, the reaction yield and the efficiency, the amount of acid used should preferably be 0.9 mol or more, and more preferably from 0.9 to 3 mol, and most preferably from 1 to 1.5 mol, per 1 mol of the aminoalcohol (1). If the amount of acid is less than 0.9 mol, the reaction does not proceed satisfactorily, and the yield of the tetrahydropyranyloxyamine (2) may be unsatisfactory.

There are no particular restrictions on the amount of 3,4-dihydro-2H-pyran to be used, although from the viewpoint of improving the reaction yield, amounts from 1 to 5 mol are preferred, and amounts from 1 to 2 mol per 1 mol of the aminoalcohol are particularly preferred.

In the production process of the present invention, first, the aminoalcohol (1) is reacted with an acid, and the produced aminoalcohol salt is then reacted with 3,4-dihydro-2H-pyran. There is no need to separate the aminoalcohol salt from the reaction solution produced by the reaction of the aminoalcohol (1) and the acid, and the aminoalcohol salt in the liquid can react with the 3,4-dihydro-2H-pyran by simply adding the 3,4-dihydro-2H-pyran to the reaction solution.

The reaction is conducted in DMF or DMSO as a reaction solvent. In view of preventing insolubility of the product salt and increasing the reaction rate these aprotic polar organic solvents, i.e. dimethylformamide (DMF) and dimethylsulfoxide (DMSO) are used. In view of improving the reaction rate; the reaction yield and the efficiency, the amount of the polar solvent used should preferably be from 0.1 to 50 parts by mass, and more preferably from 0.5 to 3 parts by mass, per 1 part by mass of the aminoalcohol (1).

Furthermore, in view of promoting the reaction while suppressing the generation of by-products, the reaction temperature should preferably be within a range from -20 to 100°C, with temperatures from -10 to 70°C being more preferred. In addition, in view of increasing the rate of reaction, the reaction temperature from 20 to 50°C are particularly preferred.

The reaction pressure should preferably be an absolute pressure from 50 kPa to 5 MPa, and more preferably from 50 kPa to 1 MPa, and most preferably from 80 kPa to 120 kPa.

In view of increasing the reaction yield, the reaction time should preferably be from 0.1 to 40 hours after adding the aminoalcohol (1), with reaction times from 0.1 to 2 hours being particularly preferred. The reaction described above then results in the formation of a salt of the tetrahydropyranyloxyamine (2).

Subsequently, the salt of the tetrahydropyranyloxyamine (2) is reacted with alkali to produce the tetrahydropyranyloxyamine (2). Examples of suitable alkali materials include sodium hydroxide, potassium hydroxide, sodium carbonate, sodium hydrogencarbonate, potassium carbonate and potassium hydrogencarbonate. The amount of alkali used should preferably be 1 to 2 equivalents relative to the acid used. The reaction should preferably be conducted by adding the solution containing the salt of the tetrahydropyranyloxyamine (2) dropwise to an aqueous solution of the alkali material. The reaction temperature should preferably be within a range from -50 to 120°C, with temperatures from -10 to 40°C being particularly preferred. There are no particular restrictions on the reaction time.

The reaction of the salt of the tetrahydropyranyloxyamine (2) and the alkali material should preferably be conducted after concentrating the reaction solution obtained after the reaction of the aminoalcohol salt with 3,4-dihydro-2H-pyran, or after extracting the salt of the tetrahydropyranyloxyamine (2) from the reaction mixture of the salt and by-products by adding a poor solvent. Concentration can be conducted in accordance with normal methods.

The aforementioned poor solvent should preferably be a solvent which results in a separation into two layers when added to the reaction solvent. Suitable examples of such poor solvents include aliphatic hydrocarbons such as n-pentane, n-hexane, 1-hexene, n-octane, isooctane and n-decane; alicyclic hydrocarbons such as cyclohexane and cycloheptane; aromatic hydrocarbons such as benzene, toluene and xylene; ether-based solvents such as diethyl ether, isopropyl ether, n-butyl ether, methyl-t-butyl ether and anisole; ester-based solvents such as ethyl acetate, butyl acetate and amyl acetate; and ketone-based solvents such as methyl ethyl ketone, methyl isopropyl ketone and methyl isobutyl ketone. The amount of poor solvent added should preferably be from 0.5 to 50 ml per 1 ml of the reaction solvent, and in view of factors such as the efficiency, amounts from 0.5 to 3 ml per 1 ml of the reaction solvent are particularly preferred.

After the addition of the poor solvent, the mixture is preferably stirred for 5 to 60 minutes, and subsequently, the mixture is preferably stood for 5 to 60 minutes. As a result, neutral compounds and the like derived from the 3,4-dihydro-2H-pyran transfer into the poor solvent layer, while the salt of the tetrahydropyranyloxyamine (2) remains in the reaction solvent layer, and therefore, the salt of the tetrahydropyranyloxyamine (2) and the aforementioned neutral components can be separated from the reaction mixture.

After the reaction of the salt of the tetrahydropyranyloxyamine (2) and the alkali material, the tetrahydropyranyloxyamine (2) can be obtained by extracting from the reaction solution with another extraction solvent. As examples of the extraction solvent, the above-described poor solvents and the like are suitable.

### EXAMPLES

The present invention is described in more detail by examples, however, the present invention is not limited to the examples. Structures of compounds in the following examples were determined by nuclear magnetic resonance (NMR), and the reaction yields and chemical purities were analyzed by gas layer chromatography (GLC) analysis.

### (Example 1)

Production of 3-[(tetrahydro-2H-pyran-2-yl)oxy]-1-propanamine

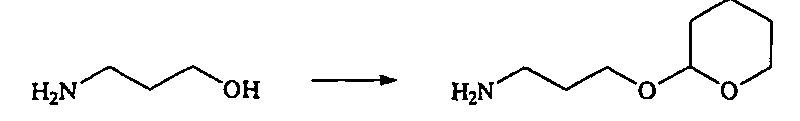

In a glass lined reaction vessel equipped with a stirrer, a feed pump, a thermometer and a cooling jacket, 21.93 g of dimethylformamide was charged under atmospheric pressure and 10.57 g of methanesulfonic acid was then added dropwise over 30 minutes with constant stirring and the internal temperature maintained at 20 to 30°C. Subsequently, with the internal temperature still maintained at 20 to 30°C, 7.51 g of 3-aminopropanol (chemical purity 99.9%) was added dropwise. Subsequently, with the internal temperature maintained at 20 to 25°C, 10.09 g of 3,4-dihydro-2H-pyran was then added dropwise over one hour. The reaction solution was subsequently aged for one hour at 20 to 30°C in order to complete the tetrahydropyranylation. In the reaction solution, 31.8 g of n-hexane was added and stirred for 15 minutes, and then the mixture was stood for 30 minutes, and as a result, the reaction solution was separated into an n-hexane layer (upper layer) and a dimethylformamide layer (lower layer).

Subsequently, in the reaction vessel in which 52.8 g of a 10% aqueous sodium hydroxide solution cooled at 10°C or lower was charged, the dimethylformamide layer (lower layer) was added dropwise with the temperature maintained at 10°C or lower. Furthermore, the reaction mixture was washed 9 times with 35.5 g of methyl-t-butyl ether (MTBE) and then concentrated under reduced pressure to produce an oily compound shown above (3-[(tetrahydro-2H-pyran-2-yl)oxy]-1-propanamine) with a yield of 74.9%. Compound yield: 13.96 g (chemical purity 85.4%, diastereomeric mixture) Compound ¹H-NMR (CDCL₃) ä ppm: 1.45-1.65 (8H, m), 2.60-2.65 (2H, t), 3.34-3.44 (2H, m), 3.64-3.74 (2H, m), 4.48-4.54 (1H, m)

### (Example 2)

Production of 3-methyl-4-[(tetrahydro-2H-pyran-2-yl)oxy]-1-butanamine

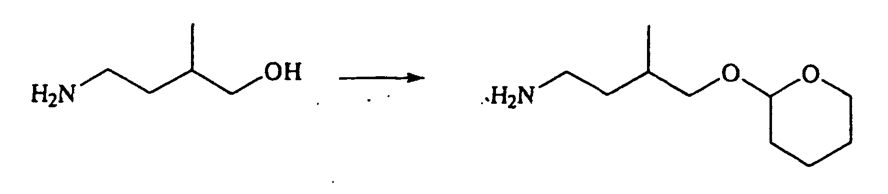

In a glass reaction vessel equipped with a stirrer, a feed pump, a thermometer and a cooling jacket, 4 g of dimethylsulfoxide was charged under atmospheric pressure and 2.05 g of methanesulfonic acid was added dropwise over 10 minutes with constant stirring and the internal temperature maintained at 20 to 30°C. Subsequently, with the internal temperature still maintained at 20 to 30°C, 2.0 g of 2-methyl-4-aminobutan-1-ol (chemical purity 99%) was added dropwise over 10 minutes. Subsequently, with the internal temperature maintained at 20 to 25°C, 2.13 g of 3,4-dihydro-2H-pyran was then added dropwise over one hour. The reaction solution was subsequently aged for one hour at 20 to 30°C in order to complete the tetrahydropyranylation. In the reaction solution, 8.47 g of n-heptane was added and stirred for 15 minutes, and then the mixture was stood for 30 minutes, and as a result, the reaction solution was separated into an n-heptane layer (upper layer) and a dimethylsulfoxide layer (lower layer).

Subsequently, in the reaction vessel in which 9.37 g of a 10% aqueous sodium hydroxide solution cooled at 10°C or lower was charged, the dimethylsulfoxide layer (lower layer) was added dropwise with the temperature maintained at 10°C or lower. Furthermore, the reaction mixture was washed twice with 10.4 g of methyl-t-butyl ether (MTBE) and then concentrated under reduced pressure to produce an oily compound shown above (3-methyl-4-[(tetrahydro-2H-pyran-2-yl)oxy]-1-butanamine) with a yield of 91.5%.
Compound yield: 3.49 g (chemical purity 95.1%, moisture content 0.90 wt%, diastereomeric mixture)
Compound ¹H-NMR (CDCL₃) ä ppm: 0.93 and 0.95 (3H, d), 1.22-1.37 (1H, m), 1.52-1.84 (8H, m), 2.66-2.82 (2H, m), 3.15-3.25 (1H, m), 3.48-3.63 (2H, m), 3.81-3.88 (1H, m), 4.55-4.59 (1H, m)

### (Example 3)

### Production of (R)-3-methyl-4-[(tetrahydro-2H-pyran-2-yl)oxy]-1-butanamine

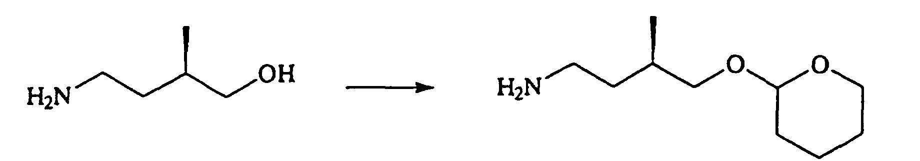

In a glass lined reaction pot equipped with a stirrer, a feed pump, a thermometer and a cooling jacket, 400 g of dimethylsulfoxide was charged under atmospheric pressure and 204.7 g of methanesulfonic acid was then added dropwise over 30 minutes with constant stirring and with the internal temperature maintained at 20 to 30°C,. Subsequently, with the internal temperature still maintained at 20 to 30°C, 200.1 g of (R)-2-methyl-4-aminobutan-1-ol (chemical purity 96%, optical purity 99.9%ee or more) was added dropwise over 35 minutes. Subsequently, with the internal temperature then maintained at 20 to 25°C, 212.7 g of 3,4-dihydro-2H-pyran was then added dropwise over one hour. The reaction solution was subsequently aged for one hour at 20 to 30°C in order to complete the tetrahydropyranylation. In the reaction solution, 846.9 g of n-heptane was added and stirred for 15 minutes, and then the mixture was stood for 30 minutes, and as a result, the reaction solution was separated into an n-heptane layer (upper layer) and a dimethylsulfoxide layer (lower layer).

Subsequently, in the reaction pot in which 940 g of a 10% aqueous sodium hydroxide solution cooled at 10°C or lower was charge, the dimethylsulfoxide layer (lower layer) was added dropwise with the temperature maintained at 10°C or lower. Furthermore, the reaction mixture was washed twice with 1,044 g of methyl-t-butyl ether (MTBE) and then concentrated under reduced pressure to produce an oily compound shown above ((R)-3-methyl-4-[(tetrahydro-2H-pyran-2-yl)oxy]-1-butanamine) with a yield of 99.9%. Compound yield: 382.0 g (chemical purity 92.4%, optical purity at least 99.9%ee, moisture content 1.28 wt%, diastereomeric mixture)

### INDUSTRIAL APPLICABILITY

According to the production process of the present invention, a tetrahydropyranyloxyamine can be produced from an aminoalcohol using a process which is both simple and produces a high yield.

## Claims

1. A production process for a tetrahydropyranyloxyamine represented by a general formula (2) shown below, wherein an aminoalcohol represented by a general formula (1) shown below is reacted with an acid to form an aminoalcohol salt, the aminoalcohol salt is reacted with 3,4-dihydro-2H-pyran to form a tetrahydropyranyloxyamine salt, and the tetrahydropyranyloxyamine salt is subsequently reacted with an alkali to form the tetrahydropyranyloxyamine.
H₂N―X―OH (1) 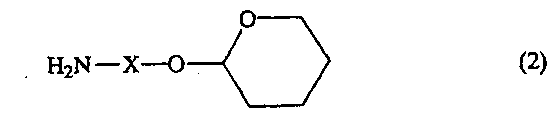
(wherein in said formula (1) and said formula (2), X represents any one of a methylene group, an ethylene group and a straight chain polymethylene group having 3 to 20 carbon atoms, and one or more hydrogen atoms of said methylene group, ethylene group or straight chain polymethylene group having 3 to 20 carbon atoms may be substituted with either one of a straight chain and a branched chain alkyl group, alkenyl group, alkynyl group, alkoxyalkyl group or alkylthioalkyl group, or a phenyl group or a halogen atom, and furthermore, one or more hydrogen atoms connected to carbon atoms not adjacent to an amino group and a hydroxy group may be substituted with a straight chain or a branched chain alkoxy group or alkenyloxy group), wherein one of dimethylformamide and dimethylsulfoxide is used as reaction solvent.

2. A production process for a tetrahydropyranyloxyamine according to claim 1, wherein said acid is either one of methanesulfonic acid and p-toluenesulfonic acid.

3. A production process for a tetrahydropyranyloxyamine according to claim 1, wherein an amount of said acid is 0.9 mol or more per 1 mol of said aminoalcohol.

4. A production process for a tetrahydropyranyloxyamine according to claim 1, wherein said aminoalcohol is either one of 3-aminopropanol and 4-amino-2-methylbutan-1-ol as represented by a general formula (3) shown below.

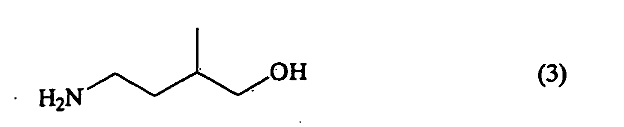

5. A production process for a tetrahydropyranyloxyamine according to claim 4, wherein said 4-amino-2-methylbutan-1-ol is optically active.

## Patentansprüche

1. Herstellungsverfahren für ein Tetrahydropyranyloxyamin der nachstehend angegebenen allgemeinen Formel (2), worin ein Aminoalkohol der nachstehend angegebenen allgemeinen Formel (1) mit einer Säure unter Bildung eines Aminoalkoholsalzes umgesetzt wird, das Aminoalkoholsalz mit 3,4-Dihydro-2H-pyran unter Bildung eines Tetrahydropyranyloxyaminsalzes umgesetzt wird und das Tetrahydropyranyloxyaminsalz danach mit einem Alkali unter Bildung des Tetrahydropyranyloxyamins umgesetzt wird:
H₂N―X―OH (1) 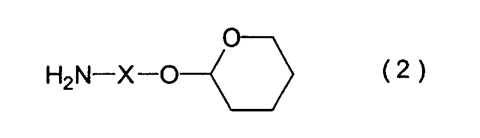
(worin in der Formel (1) und der Formel (2) X eine Methylengruppe, eine Ethylengruppe oder eine geradkettige Polymethylengruppe mit 3 bis 20 Kohlenstoffatomen ist, und ein oder mehrere Wasserstoffatome der Methylengruppe, Ethylengruppe oder geradkettigen Polymethylengruppe mit 3 bis 20 Kohlenstoffatomen durch eine geradkettige oder verzweigtkettige Alkylgruppe, Alkenylgruppe, Alkinylgruppe, Alkoxyalkylgruppe oder Alkylthioalkylgruppe, oder eine Phenylgruppe oder ein Halogenatom substituiert sein kann, und ferner ein oder mehrere Wasserstoffatome, die mit Kohlenstoffatomen verbunden sind, die nicht zu einer Aminogruppe und einer Hydroxygruppe benachbart sind, durch eine geradkettige oder verzweigtkettige Alkoxygruppe oder Alkenyloxygruppe substituiert sein können), worin eines unter Dimethylformamid und Dimethylsulfoxid als Reaktionslösungsmittel verwendet wird.

2. Herstellungsverfahren für ein Tetrahydropyranyloxyamin gemäss Anspruch 1, worin die Säure eine unter Methansulfonsäure und p-Toluolsulfonsäure ist.

3. Herstellungsverfahren für ein Tetrahydropyranyloxyamin gemäss Anspruch 1, worin die Menge der Säure 0,9 mol oder mehr pro 1 mol des Aminoalkohols ist.

4. Herstellungsverfahren für ein Tetrahydropyranyloxyamin gemäss Anspruch 1, worin der Aminoalkohol entweder 3-Aminopropanol oder 4-Amino-2-methylbutan-1-ol, wie durch die nachstehend gezeigte allgemeine Formel (3) dargestellt, ist:

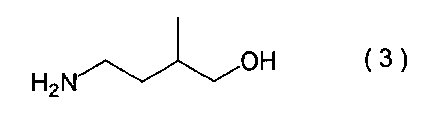

5. Herstellungsverfahren für ein Tetrahydropyranyloxyamin gemäss Anspruch 4, worin das 4-Amino-2-methylbutan-1-ol optisch aktiv ist.

## Revendications

1. Procédé de production pour une tétrahydropyranyloxyamine représentée par une formule générale (2) montrée plus bas, dans lequel un aminoalcool représenté par la formule générale (1) montrée plus bas est mis à réagir avec un acide afin de former un sel d'aminoalcool, le sel d'aminoalcool est mis à réagir avec un 3,4-dihydro-2H-pyrane afin de former un sel de tétrahydropyranyloxyamine, et le sel de tétrahydropyranyloxyamine est ultérieurement mis à réagir avec un produit alcalin afin de former la tétrahydropyranyloxyamine.
H₂N`―X―OH (1) 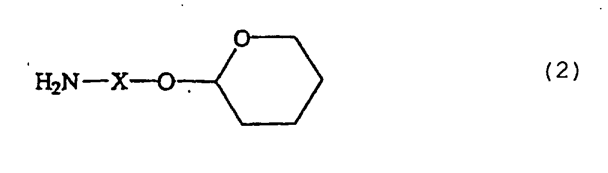
(où dans ladite formule (1) et ladite formule (2), X représente l'un quelconque de un groupe méthylène, un groupe éthylène et un groupe polyméthylène à chaîne linéaire ayant 3 à 20 atomes de carbone, et un ou plusieurs atomes d'hydrogène dudit groupe méthylène, groupe éthylène ou groupe polyméthylène à chaîne linéaire ayant 3 à 20 atomes de carbone peuvent être substitués avec soit l'un de un groupe alkyle à chaîne linéaire et à chaîne ramifiée, un groupe alcényle, un groupe alcynyle, un groupe alcoxyalkyle ou un groupe alkylthioalkyle, soit un groupe phényle ou un atome d'halogène, et en outre, un ou plusieurs atomes d'hydrogène liés à des atomes de carbone non adjacents à un groupe amino et à un groupe hydroxyle peuvent être substitués avec un groupe alcoxy ou groupe alcényloxy à chaîne linéaire ou à chaîne ramifiée), où l'un du diméthylformamide et du diméthylsulfoxyde est utilisé en tant que solvant de réaction.

2. Procédé de production pour une tétrahydropyranyloxyamine selon la revendication 1, dans lequel ledit acide est l'un ou l'autre de l'acide méthanesulfonique et de l'acide p-toluènesulfonique.

3. Procédé de production pour une tétrahydropyranyloxyamine selon la revendication 1, dans lequel une quantité dudit acide est de 0,9 mole ou plus pour 1 mole dudit aminoalcool.

4. Procédé de production pour une tétrahydropyranyloxyamine selon la revendication 1, dans lequel ledit aminoalcool est l'un ou l'autre du 3-aminopropanol et du 4-amino-2-méthylbutan-1-ol comme représenté par une formule générale (3) montrée plus bas.

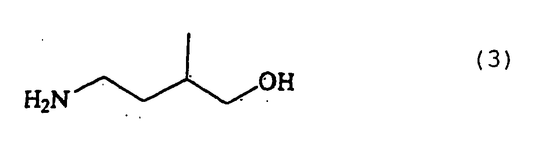

5. Procédé de production pour une tétrahydropyranyloxyamine selon la revendication 4, dans lequel ledit 4-amino-2-méthylbutan-1-ol est optiquement actif.
